# EUROPÄISCHE PATENTSCHRIFT

(11) **EP 3 103 484 B1**
(45) Veröffentlichungstag und Bekanntmachung des Hinweises auf die Patenterteilung: **31.07.2019**
(21) Anmeldenummer: 16164380.4
(22) Anmeldetag: 08.04.2016
(51) Int. Cl.: A61L 27/36, A61L 27/24

(54) **VERFAHREN ZUR AUFBEREITUNG VON BIOLOGISCHEM GEWEBE**
METHOD FOR THE TREATMENT OF BIOLOGICAL TISSUE
PROCEDE DE PREPARATION DE TISSUS BIOLOGIQUES

(30) Priorität: 08.06.2015 DE 102015108952
(43) Veröffentlichungstag der Anmeldung: 14.12.2016
(73) Patentinhaber: Biotronik AG, 8180 Bülach (CH)
(72) Erfinder: RZANY, Alexander, 90449 Nürnberg (DE); ERDBRÜGGER, Wilhelm, 78467 Konstanz (DE)
(74) Vertreter: Randoll, Sören

(56) Entgegenhaltungen:
- EP-A1- 2 832 379
- KR-A- 20120 002 379
- KIM GI BEOM ET AL: "Novel self-expandable, stent-based transcatheter pulmonic valve: A preclinical animal study", INTERNATIONAL JOURNAL OF CARDIOLOGY, Bd. 173, Nr. 1, 20. Februar 2014 (2014-02-20), Seiten 74-79, XP028833913, ISSN: 0167-5273, DOI: 10.1016/J.IJCARD.2014.02.005

## Beschreibung

Die vorliegende Erfindung betrifft ein Verfahren zur Aufbereitung von Gewebe für medizinische Anwendungen, insbesondere von Gewebe zur Verwendung für eine künstliche Herzklappe.

Die vorliegende Erfindung ist zwar zur Aufbereitung derartigen Gewebes besonders geeignet, jedoch nicht auf diese Anwendung beschränkt. Die vorliegende Erfindung ist auch für die Aufbereitung von Blutgefäßen, Knochen, Knorpeln, Bändern, Cornea oder dergleichen anwendbar.

Mechanische Herzklappenprothesen und die damit verbundenen begleitenden Medikationen können erhebliche Einschränkungen für die Patienten mit sich bringen. Patienten mit einer mechanischen Herzklappe müssen sich lebenslang einer Antikoagulationsbehandlung unterziehen und unterliegen damit dauerhaft einer erhöhten Gefährdung durch thromboembolische Komplikationen als auch durch Blutungen.

Um diese Nachteile und Schwierigkeiten zu umgehen, wurden biologische Herzklappenprothesen auf der Basis von humanem Gewebe (als Allo- oder Homograft) oder tierischem Gewebe (als Xenograft) entwickelt. Bei der Entwicklung von biologischen Herzklappenprothesen, insbesondere bei solchen Prothesen mit Klappensegeln aus biologischem Material tierischen Ursprungs (sog. Xenografts), kann auf eine dauerhafte Antikoagulation verzichtet werden. Allerdings neigen biologische Herzklappenprothesen zur Kalzifizierung, wobei die Verkalkung zum Funktionsverlust der Prothese führen kann. Es konnte gezeigt werden, dass trotz aller Sorgfalt und Vorsicht bei der Aufbereitung des biologischen Materials bereits geringe Rückstände von nicht vollständig denaturierten Proteinen oder Zellen nach der Implantation zu einer biologischen Antwort führen können.

Im Ergebnis werden frühzeitige Kalzifizierungen des biologischen Klappenmaterials beobachtet. Die biologische Herzklappenprothese versagt und muss gegebenenfalls ausgetauscht werden. Neben der Kalzifizierung stellt auch die andauernde mechanische Belastung der Prothesen ein Problem dar, so dass biologische Herzklappenprothesen meist eine geringere Lebensdauer aufweisen als mechanische Herzklappenprothesen.

Die aus dem biologischen Gewebe geformte Herzklappe wird zumeist in einem Grundkörper (z.B. ein festes Kunststoffgerüst oder ein selbstexpandierender Stent) befestigt und dieser wird an der Position der natürlichen Klappe implantiert. Die vorliegende Erfindung beschreibt ein Verfahren zur Aufbereitung eines derartigen Gewebes zum Einsatz in einer Herzklappenprothese, die an Stelle einer natürlichen Herzklappe implantiert werden soll.

Das Ausgangsgewebe muss vor der Implantation einer Prozedur mit mehreren Schritten unterzogen werden, um als bearbeitetes Gewebe implantiert zu werden. Dabei wird das Gewebe möglichst so modifiziert, dass es vom Körper nicht als Fremdgewebe erkannt wird, nicht verkalkt und eine möglichst lange Lebensdauer hat. Im Wesentlichen umfasst ein derartiges Verfahren zur Aufbereitung von Gewebe mindestens zwei Hauptschritte mit mehreren zwischengeschalteten Spülprozessen, wobei das Ausgangsgewebe vor den eigentlichen Behandlungsschritten gründlich gereinigt und aufbereitet werden muss.

Der erste wesentliche Aufbereitungsschritt ist die sogenannte Dezellularisierung des Gewebes. In diesem Schritt werden Zellmembranen, intrazelluläre Proteine, Zellkerne und andere Zellbestandteile möglichst vollständig aus dem Gewebe entfernt, um eine möglichst reine extrazelluläre Matrix zu erhalten. Im Gewebe verbleibende Zellen und Zellbestandteile wären insbesondere potente Ausgangspunkte für eine unerwünschte Kalzifizierung des biologischen Implantatmaterials. Die Dezellularisierung als Waschschritt soll dabei möglichst so schonend durchgeführt werden, dass die Struktur und die Kollagenfasern in der extrazellulären Matrix möglichst unbeeinflusst bleiben, während andererseits gründlich alle darin enthaltenen Zellen aus dem Gewebe entfernt werden. Gereinigte, aber ansonsten unbeeinflusste, intakte Kollagenfasern sind bevorzugt, da diese die mechanische Stabilität des weiterverarbeiteten Gewebes sicherstellen.

Der zweite wesentliche Aufbereitungsschritt besteht in einer Vernetzung, insbesondere der Kollagenfasern, des Gewebes. Nach der Dezellularisierung sind möglichst alle Zellbestandteile aus dem Gewebe entfernt und das biologische Material besteht fast ausschließlich aus der extrazellulären Matrix. Bei Herzbeutelgewebe wird die extrazelluläre Matrix dabei vorwiegend aus Kollagenfasern gebildet. Um ein biologisches Material mit möglichst optimalen mechanischen Eigenschaften zu erreichen und Abwehrreaktionen des empfangenden Körpers zu verhindern, werden die Kollagenfasern mittels eines geeigneten Vernetzungsmittels durch den Einbau chemischer Bindungen quervernetzt. Das Vernetzungsmittel bindet an die Aminogruppen der Kollagenfasern an und bildet chemisch stabile Verbindungen zwischen Kollagenfasern. So entsteht aus dem dreidimensional angeordneten Kollagenfasern ein langzeitstabiles biologisches Material, welches zudem nicht mehr als biologisches Fremdmaterial erkannt wird. Durch die dreidimensionale Vernetzung bzw. Verknüpfung der einzelnen Kollagenfasern über das Vernetzungsmittel werden die Stabilität und die Beanspruchbarkeit des Gewebes deutlich erhöht. Dies ist insbesondere bei einem Einsatz als Gewebe einer Herzklappe entscheidend, wo das Gewebe über dem Sekundentakt als Klappe öffnen und schließen muss.

Die oben erwähnten Kalzifizierungen können jedoch trotz sorgfältig durchgeführter Dezellularisierung auftreten und haben dabei unter anderem ihren Ursprung in gegen Galaktose-α-1,3-galaktose-β-1,4-*N*-acetylglucosamin-Epitope (α-Gal-Epitope auf der Oberfläche des implantierten Gewebes) gebildeten Antikörpern. α-Gal-Epitope können dabei zu starken Immunreaktionen führen, die die Kalzifizierung fördern. Die Konzentration von α-Gal-Epitopen auf der Oberfläche könnte grundsätzlich durch harsche Dezellularisierungsbedingungen verringert werden. Dies würde jedoch einen deutlich negativen Einfluss auf die mechanischen Eigenschaften des Klappenmaterials ausüben. Um Kalzifizierungen zu minimieren und Gewebe mit deutlich verbesserten mechanischen Eigenschaften bereitzustellen, wäre es daher wünschenswert, Gewebe bereitzustellen, das einer schonenden Dezellularisierung unterzogen wurde und bei dem α-Gal-Epitope auf der Oberfläche des Gewebes nach Möglichkeit vollständig entfernt wurden. KR 20120002379 A und Kim Gi Beom et al., (Novel self-expandable, stent-based transcatheter pulmonic valve: A preclinical animal study, International Journal of Cardiology, Bd. 173, Nr. 1, Seiten 74-79) offenbaren Dezellularisierung von biologischen Klappen in Gegenwart von Detergenz und α-Galactosidase. Verfahren, die effizienter und/oder mit kleineren Mengen an α-Galactosidase durchgeführt werden können, wären jedoch wünschenswert.

Im Lichte des oben angeführten wird ein Verfahren nach Anspruch 1 vorgeschlagen.

Insbesondere wird ein Verfahren zur Aufbereitung von biologischen Gewebe vorgeschlagen, das es ermöglicht, Zellbestandteile und α-Gal-Epitope gründlich aber schonend vom Gewebe in der Weise zu entfernen, dass eine anschließende Vernetzung ein mechanisch stabiles und langlebiges Gewebe erzeugt, welches insbesondere zu einem Einsatz als Gewebe einer künstlichen Herzklappe geeignet ist und dabei eine signifikant reduzierte Neigung zur Kalzifizierung aufweist. Dies wird durch das hierin vorgeschlagene Verfahren bereitgestellt.

Im Lichte des oben angeführten wird ein Verfahren zur Aufbereitung von Gewebe für medizinische Anwendungen, insbesondere von Gewebe zur Verwendung für eine künstliche Herzklappe vorgeschlagen. Es wird ein Verfahren vorgeschlagen, dass einen Schritt der Dezellularisierung des Gewebes mittels eines Detergens umfasst. Durch die Dezellularisierung werden die Zellbestandteile aus dem Gewebe entfernt und das biologische Material besteht fast ausschließlich aus der extrazellulären Matrix. Ferner wird ein Schritt der Behandlung des Gewebes mit mindestens einer α-Galaktosidase vorgeschlagen. Durch die Behandlung mit mindestens einer α-Galaktosidase können α-Gal-Epitope auf der Oberfläche des Gewebes entfernt und das Risiko einer späteren Kalzifizierung signifikant verringert oder sogar minimiert werden. In einer bevorzugten Ausführungsform wird das Gewebe mit einer α-Galaktosidase behandelt. Es ist aber ebenfalls möglich eine Kombination von α-Galaktosidasen zu verwenden. Dies bedeutet, dass die in Kombination verwendeten α-Galaktosidasen eine unterschiedlicher Struktur und/oder Herkunft aufweisen; das heißt dass die α-Galaktosidasen in einem unterschiedlichen Lebewesen produziert wurden und/oder eine unterschiedliche Struktur aufweisen.

Es wird ferner vorgeschlagen, dass das vorgeschlagene Verfahren einen Schritt der Vernetzung der extrazellulären Matrix des Gewebes mittels eines geeigneten Vernetzungsmittels umfasst. In einer bevorzugten Ausführungsform liegt die extrazelluläre Matrix in Form von Kollagenfasern vor. Das Vernetzungsmittel bindet an die Aminogruppen der Kollagenfasern an und bildet chemisch stabile Verbindungen zwischen den Kollagenfasern. So entsteht aus dem dreidimensional angeordneten Kollagenfasern ein langzeitstabiles biologisches Material, welches zudem nicht mehr als biologisches Fremdmaterial erkannt wird. Ferner wird das Gewebe durch die Vernetzung mechanisch stabiler.

α-Galaktosidasen, auch α-D-Galactosidgalactohydrolase, E.C. 3.2.1.22, sind Enzyme, die in der Lage sind, die Hydrolyse von Galactosylresten der nichtreduzierenden Enden einer Vielzahl von Oligisacchariden und Polysacchariden, sowie von Galactolipiden und Glycoproteinen zu katalysieren. In Bezug auf Gewebe können α-Galaktosidasen dazu eingesetzt werden, α-1,3-Galaktosylreste auf und in dem Gewebe zu entfernen. Es konnte dabei gefunden werden, dass durch Behandlung mit α-Galaktosidasen α-Gal-Epitope effektiv von der Oberfläche des Gewebes entfernt werden können, wodurch Immunreaktionen und Kalzifizierungen verringert werden können.

α-Galaktosidasen können je nach Herkunft in ihrer Aufgabe, strukturell und in Ihrer Wirkung signifikant divergieren. Dies ist stark damit verbunden, dass eine Vielzahl von Organismen α-Galaktosidasen produziert, wie zum Beispiel Archaeen, Bakterien, Pilze, Pflanzen oder Tiere. Eine mögliche Gruppierung von α-Galaktosidasen kann in der Aufgabe im jeweiligen Organismus liegen. Dabei kann zum Beispiel eine Gruppierung durch die pH-Abhängigkeit der Enzymaktivität gegeben sein. Die Erfinder haben herausgefunden, dass sich nicht alle α-Galaktosidasen gleich gut für die Behandlung von Gewebe für die Verwendung in Herzklappen eignen. Vielmehr haben die Erfinder überraschend gefunden, dass bestimmte α-Galaktosidasen insbesondere für die Verwendung im hierin vorgeschlagenen Verfahren geeignet sind.

In einer bevorzugten Ausführungsform wird für das hierin vorgeschlagene Verfahren die Verwendung von alkalischen α-Galaktosidasen vorgeschlagen. Alkalische α-Galaktosidasen zeichnen sich dadurch aus, dass diese im alkalischen Medium eine hohe oder ihre höchste enzymatische Aktivität aufweisen und dabei ferner eine hohe Substratspezifität aufweisen. Die Verwendung von alkalischen α-Galaktosidasen ist vorteilhaft, da es dadurch ermöglicht wird, parallel zu den alkalischen α-Galaktosidasen auch DNasen und RNasen zu verwenden. DNasen und RNasen dienen der Entfernung von restlichen Ribonukleinsäuren aus dem Gewebe, die auch zu einer Kalziumbindung beitragen können. Durch Kombination von α-Galaktosidasen mit DNasen und/oder RNasen kann somit ein noch verbesserter Schutz gegenüber Kalzifizierung erreicht werden. Insbesondere sind α-Galaktosidasen bevorzugt, die im leicht alkalischen, insbesondere in einem pH-Bereich von 7.1 bis 8.0, weiter bevorzugt in einem pH-Bereich von 7.2 bis 7.8 und am meisten bevorzugt, die in einem pH-Bereich von 7.3 bis 7.6 die höchste spezifische Enzymaktivität zeigen. Bevorzugte alkalische α-Galaktosidasen stammen von Arabidopsis thaliana, Cucumis melo, Cucumis sativus, Oryza sativa, beispielsweise der Japonica-Gruppe, Pisum sativum, Solanum Lycopersicum, Tetragonia tetragonioides und Zea mays.

In einer weiteren bevorzugten Ausführungsform werden für das vorgeschlagene Verfahren α-Galaktosidasen aus der Familie GH-36 verwendet. Es hat sich gezeigt, dass Vertreter der Familie GH-36 hoch effizient α-Gal-Epitope auf Gewebe entfernen können. Die Erfinder konnten überraschend feststellen, dass α-Galaktosidasen aus der Familie GH-36 deutlich schneller und bei geringerer Konzentration α-Gal-Epitope auf Gewebe entfernen können als Vertreter anderer GH- Familien.

In einer weiteren bevorzugten Ausführungsform werden für das vorgeschlagene Verfahren α-Galaktosidasen aus der Familie GH-36, Subgruppe II verwendet. Vertreter dieser Subgruppe haben sich als besonders geeignet gezeigt α-Gal-Epitope auf Gewebe effizient entfernen können. Bevorzugte α-Galaktosidasen aus der Familie GH-36, Subgruppe II werden von folgenden Quellen bezogen, ausgewählt aus der Gruppe umfassend oder bestehend aus Oryza sativa der Japonica-Gruppe, Cucumis melo, Bifidobacterium breve C50, Sulfolobus solfactarius und Sulfolobus tokodaii. In einer besonders bevorzugten Ausführungsform des hierin vorgeschlagenen Verfahrens stammt die α-Galaktosidase von Cucumis melo.

Es konnte insbesondere gezeigt werden, dass α-Galaktosidasen von Cucumis melo in der Lage sind α-Gal-Epitope auf Gewebe spezifischer zu entfernen als beispielsweise α-Galaktosidasen der grünen Kaffeebohne (green coffee bean, GCB) oder der sauren Variante von Aspergillus niger.

Wie oben dargelegt kann das vorgeschlagene Verfahren zusätzliche Schritte neben der Behandlung von Gewebe mit α-Galaktosidasen aufweisen. Dies ist insbesondere hinsichtlich der Erlangung eines Gewebes vorteilhaft, dass sowohl ausgezeichnete mechanische Eigenschaften als auch geringe bis keine Neigung zur Kalzifizierung aufweist.

Gemäß dem vorgeschlagenen Verfahren wird das Gewebe einer Dezellularisierung mit einem Detergens unterzogen, wobei bevorzugt Lipopeptide als Detergens zur Dezellularisierung eingesetzt werden. Gemäß dem vorliegenden Vorschlag werden β-Hydroxy-Fettsäure- oder β-Amino-Fettsäure-haltige Peptide, Lipopeptide, nicht zur Konditionierung sondern als Detergens zur Dezellularisierung verwendet. ES hat sich gezeigt, dass die Lipopeptide hervorragende Ergebnisse bei der Dezellularisierung von Gewebe zeigen. Das Gewebe wird deutlich schonender von Zellbestandteilen befreit. Die Struktur der extrazellulären Matrix bleibt bei der Verwendung eines oben genannten Detergens zur Dezellularisierung sehr gut erhalten, und bildet eine verbesserte Ausgangsbasis für eine erfindungsgemäße Behandlung mit α-Galaktosidasen wie hierin beschrieben. Ein geeignetes Detergens für das vorliegende Verfahren enthält somit mindestens ein Lipopeptid mit amphiphilen Eigenschaften, bestehend aus einer hydrophilen Grundstruktur und einer hydrophoben Seitenkette. Dadurch lässt sich durch den bevorzugt nachfolgenden Vernetzungsschritt ein Gewebe erzielen, was gegenüber dem Stand der Technik deutlich verbesserte mechanische Eigenschaften aufweist und sich somit insbesondere zur Verwendung in einer Herzklappenprothese eignet. Zusammen mit einem Behandlungsschritt mit α-Galaktosidasen wie hierin beschrieben, kann Gewebe zur Verfügung gestellt werden, das deutlich verbesserte mechanische Eigenschaften aufweist und zu einem minimierten Maße zu Kalzifizierung und Immunreaktionen neigt. Wird das Gewebe in ein Implantat eingebracht wie beispielsweise eine künstliche Herzklappe, zeichnen sich diese durch überaus gute mechanische Stabilität und eine lange Lebensdauer aus.

Besonders bevorzugt enthält das Detergens zur Dezellularisierung ein zyklisches Lipoheptapeptid, insbesondere Surfactin. In dieser bevorzugten Ausgestaltung der Erfindung wird ein Surfactin enthaltendes Detergens zur Dezellularisierung verwendet. Insbesondere enthält das Detergens Surfactin mit einer zyklischen Struktur wie nachfolgend wiedergegeben:

(CH₃)₂-CH-(CH₂)ₙ→CH-CH₂-CO→L-Glu→L-Leu→D-Leu→L-Val→L-Asp→D-Leu→L-Leu→O

Dabei ist n=8-12, vorzugsweise 9, und Glu, Leu, Val, Asp stehen für die Aminosäuren Glutaminsäure, Leucin, Valin und Asparginsäure.

Ebenso vorteilhaft sind Lipopeptide wie Daptomycin, Caspofungin, Arthrofactin, Echinocandine, Iturine, Syringomycine, Syringopeptide und/oder Polymyxine. Zweckmäßigerweise enthält das Detergens mindestens ein Lipopeptid ausgewählt aus der Liste: Surfactin, Daptomycin, Caspofungin, Arthrofactin oder der Gruppe der Echinocandine, Iturine, Syringomycine, Syringopeptide, Polymyxine.

Besonders bevorzugt besteht das Detergens aus einer Pufferlösung, besonders bevorzugt einer Phosphatpufferlösung, zweckmäßigerweise bei pH 7.4, die das Lipopeptid, insbesondere Surfactin, mit einer Konzentration von 100 mg/l bis 2000 mg/l, bevorzugt 500 mg/l bis 700 mg/l, besonders bevorzugt von 600 mg/l, enthält. Dabei ist der Einsatz von Dulbecco's Phosphat gepufferter Salzlösung (DPBS) ohne Kalzium und Magnesium als Trägerlösung für das Detergens Surfactin besonders vorteilhaft. Andere biologische Pufferlösungen, wie zum Beispiel Tris(hydroxymethyl)-aminomethan (TRIS)- oder 2-(4-(2-Hydroxyethyl)- 1-piperazinyl)-ethansulfonsäure (HEPES)-gepufferte Lösungen, sind ebenfalls zweckmäßig.

Vorteilhafterweise wird das Gewebe während des hierin vorgeschlagenen Verfahrens vor und besonders bevorzugt nach der Dezellularisierung zumindest einmal, bevorzugt mehrmals, mit einem geeigneten Lösungsmittel, insbesondere einer gepufferten Salzlösung und/oder einer Alkohollösung, gespült.

Das hierin vorgeschlagene Verfahren kann zudem einen Vernetzungsschritt umfassen. Das Vernetzungsmittel enthält bevorzugt Glutaraldehyd. In alternativen Ausgestaltungen der Erfindung enthält das Vernetzungsmittel Carbodiimid, Aldehyde, wie beispielsweise Formaldehyd, Glutaraldehyd-Acetale, Acyl-Azide, Cyanimid, Genepin, Tannin, Pentagalloyl-Glukose, Phytat, Proanthocyanidin, Reuterin und/oder Epoxidverbindungen.

Besonders vorteilhaft sind dabei gepufferte Natriumchloridlösungen und/oder eine Ethanollösung.

Für das vorgeschlagene Verfahren können grundsätzlich alle Arten von Gewebe vom Säugetier, einschließlich vom Menschen, verwendet werden. Bevorzugt ist nicht-humanes Gewebe. Insbesondere eignen sich solche Gewebe, die als Klappenmaterial in einer Herzklappe verwendet werden können. Bevorzugt sind dabei Perikardgewebe und Herzklappen, aber auch Haut, Bandgewebe, Sehnen, Bauchfell, Dura mater, Tela submucosa, insbesondere des Magen-Darm-Traktes, oder Rippenfell. Bei Herzklappen können alle Klappen verwendet werden, also Aorten-, Pulmonal-, Mitral- und Trikuspidalklappen. Des Weiteren sind bevorzugt Perikardgewebe vom Schwein, Schaf, Ziege, Pferd, Krokodil, Känguru, Strauß und vom Rind.

Es wird ferner die Verwendung einer α-Galaktosidase wie hierin beschrieben zur Behandlung von biologischem Gewebe vorgeschlagen, insbesondere von biologischem Gewebe für Herzklappenprothesen. Insbesondere wird die Verwendung von alkalischen α-Galaktosidasen vorgeschlagen. Ferner wird die Verwendung von α-Galaktosidasen der Familie GH-36, bevorzugt der Subgruppe II, und weiter bevorzugt von Cucumis melo vorgeschlagen.

Besonders vorteilhaft ist die Verwendung einer α-Galaktosidase wie hierin beschrieben zur Behandlung von biologischem Gewebe in Verbindung mit der Verwendung einer mindestens ein Lipopeptid, insbesondere Surfactin, Daptomycin, Caspofungin, Arthrofactin, ein Echinocandin, ein Iturin, ein Syringomycin, ein Syringopeptid und/oder ein Polymyxin enthaltenden Lösung als Detergens zur Dezellularisierung von biologischem Gewebe. Eine solche Kombination von Behandlungsschritten stellt Gewebe zu Verfügung, bei dem sehr schonend und umfassend zelluläres Matetrial und α-Gal-Epitope entfernt wurden.

Die vorliegende Erfindung stellt insbesondere ein Verfahren zur Aufbereitung von biologischem Gewebe zur Verfügung, welches eine gründliche und zuverlässige Dezellularisierung gewährleistet, welche gleichzeitig derart gewebeschonend durchgeführt wird, dass die mechanischen Eigenschaften des Gewebes nach Dezellularisierung, Behandlung mit α-Galaktosidase und Vernetzung deutlich gegenüber dem Stand der Technik verbessert werden.

Das erfindungsgemäße Verfahren zur Aufbereitung von biologischem Gewebe, insbesondere zur Aufbereitung von biologischem Gewebe zur Verwendung in einer Herzklappenprothese, minimiert zudem das Risiko einer Kalzifizierung des Gewebes (und damit der Prothese) im klinischen Einsatz durch die Behandlung mit α-Galaktosidasen wie hierin beschrieben. Durch die erfindungsgemäße Behandlung mit mindestens einer α-Galaktosidase, insbesondere in Verbindung mit einer Dezellularisierung mit einem Detergens wie hierin beschrieben, werden maßgeblich die Eigenschaften des Gewebes positiv beeinflusst. Ein nach dem erfindungsgemäßen Verfahren aufbereitetes Gewebe zeigt eine deutlich verbesserte mechanische Belastbarkeit und eine signifikant verringerte Neigung eine Immunreaktion auszulösen und zu kalzifizieren. Ferner weist Gewebe, das sowohl mit α-Galaktosidase behandelt wurde als auch mit einem hierin beschriebenen Detergens, vorzugsweise Surfactin, dezellularisiert wurde, nochmals verbesserte Oberflächeneigenschaften auf.

Im Folgenden soll die Erfindung anhand der Ausführungsbeispiele in den Figuren näher erläutert werden. Es wird dabei der Einfluss verschiedener α-Galaktosidasen auf die Konzentration von α-Gal-Epitopen auf der Oberfläche des Gewebes untersucht.

In einem Ausführungsbeispiel wird ein biologisches Gewebe aus porcinem Herzbeutelgewebe durch mechanische Entfernung von anhaftendem Fremdgewebe und anschließendem Spülen in isotonischer Kochsalzlösung (Fa. Fresenius-Kabi) für 2 Stunden bei 4°C gewonnen. Dieses Gewebe wird einer Dezellularisierung mit einem Detergens, bestehend aus einer DPBS-Lösung (Dulbecco's Phosphate Buffered Saline, Dulbeccos phosphatgepufferte Kochsalzlösung) ohne Kalzium/Magnesium (Fa. Lonza; DPBS w/o Ca++/Mg++; Art.-Nr. 17-512) und Surfactin (Fa. Sigma-Aldrich, Surfactin from Bacillus subtilis, Art.-Nr. F3523) in einer Konzentration von 600 mg/l, für 20 Stunden bei 37°C unterzogen.

Anschließend wird das dezellularisierte Gewebe mit α-Galaktosidasen von grünen Kaffeebohnen und von Zuckermelone (Cucumis melo) bei einer Konzentration von 5 Units pro ml, 1 Unit pro ml und 0.1 Units U pro ml für 24 Stunden versetzt. Als eine "Unit" wird die Menge an Enzym verstanden, die 1.0 µmol p-Nitrophenyl-α-D-galactosid zu p-Nitrophenol und D-Galaktose pro min bei pH 6.5 und 25 °C in DPBS hydrolisieren kann. Um eine Aussage zu treffen wie effektiv α-Gal-Epitope vom Gewebe entfernt wurden, wird die Absorption von spezifischen IgM-Antikörpern (M86) des behandelten Gewebes gemessen. Je weniger IgM-Antikörper an das Gewebe absorbiert, desto weniger α-Gal-Epitope sind im Gewebe verblieben. Ferner wurde ein Vergleich mit nicht dezellularisiertem Gewebe vorgenommen, das jedoch mit α-Galaktosidasen in der oben beschriebenen Weise behandelt wurde.

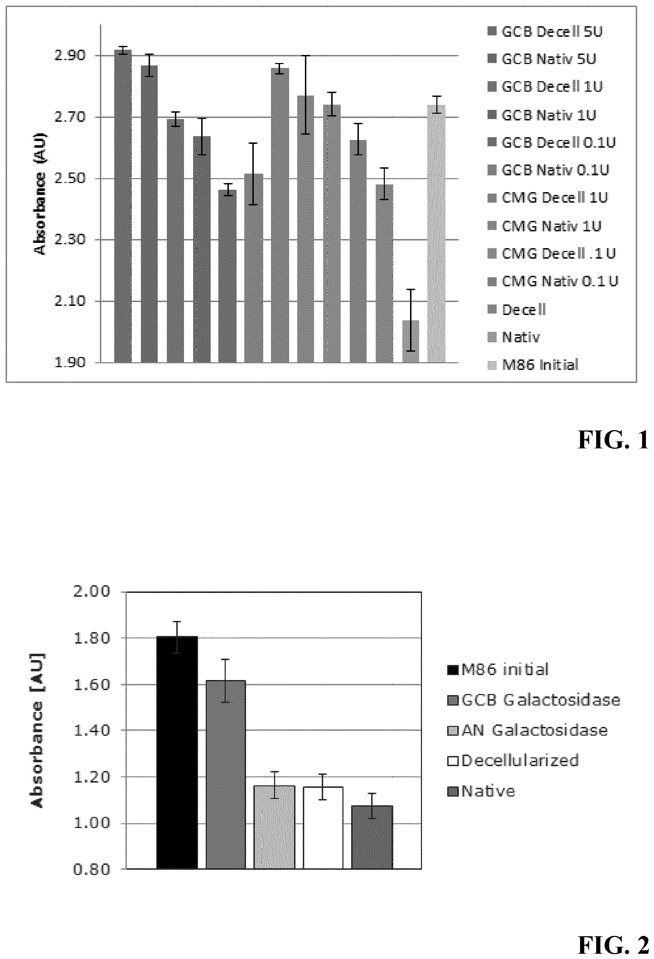

Figur 1 zeigt die Absorption von M86 Antikörpern auf dem behandelten Gewebe. In dem Diagramm werden zwei Arten von Gewebe verglichen: Natives Gewebe, das nicht dezellularisiert wurde, und dezellularisiertes Gewebe. Vergleichswerte sind auf der rechten Seite des Diagramms enthalten: M86 initial, Nativ und Decell geben die Absorptionswerte für Gewebe an, das nicht mit α-Galaktosidase behandelt wurde. Dabei zeigt das native Gewebe das höchste Maß an α-Gal-Epitopen an. M86 initial gibt diejenige Absorption an, bei der keine Absorption der Antikörper stattgefunden hat. Dieser Wert stellt den Grenzwert für Gewebe dar, auf dem keine α-Gal-Epitope mehr vorhanden sind. Aus dem Vergleich von M86 initial, Nativ und Decell wird ersichtlich, dass die Dezellularisierung bereits eine signifikante Menge an α-Gal-Epitopen entfernt (Vergleich Decell zu Nativ). Es wird aber auch deutlich, dass eine signifikante Menge an α-Gal-Epitopen auf dem Gewebe verbleibt (Vergleich Decell zu M86 initial).

Die weiteren Absorptionsdaten zeigen den Einfluss der Behandlung mit α-Galaktosidasen auf die Konzentration von α-Gal-Epitopen auf der Oberfläche des Gewebes. Die α-Galaktosidase der grünen Kaffeebohne (GCB, green coffee bean, Sigma Aldrich) bei einer Konzentration von 1 Unit pro ml vermag nicht ganz alle α-Gal-Epitope zu entfernen (Vergleich M86 initial zu GCB ^{∗} 5U). Allerdings wird durch die Verwendung von 1U der α-Galaktosidase der grünen Kaffeebohne die Konzentration von α-Gal-Epitopen auf der Oberfläche deutlich verringert (Vergleich Decell/Nativ zu GCB ^{∗} 1U). Wird die hohe Konzentration von 5 Units pro ml der α-Galaktosidase der grünen Kaffeebohne verwendet, können nahezu alle α-Gal-Epitope auf der Oberfläche des Gewebes entfernt werden (Vergleich M86 initial zu GCB ^{∗} 5U). Die außergewöhnliche Eignung der α-Galaktosidase von Cucumis melo (CMG, Cucumis melo Galaktosidase) wird an Hand von Figur 1 im Folgenden erläutert. Wird die vergleichsweise geringe Konzentration von 1 Unit pro ml verwendet, können alle α-Gal-Epitope auf der Oberfläche des Gewebes entfernt werden (Vergleich M86 initial zu CMG ^{∗} 1U). Ferner zeigt sich, dass bereits bei dezellularisiertem Gewebe eine 1/10 Unit ausreicht, um nahezu alle α-Gal-Epitope auf der Oberfläche des Gewebes zu entfernen (Vergleich M86 initial zu CMG Decell 0.1U). Bei nativem Gewebe werden bei dieser äußerst geringen Konzentration annähernd alle α-Gal-Epitope auf der Oberfläche des Gewebes entfernt (Vergleich M86 initial zu CMG Native 0.1U). Es konnte also gezeigt werden, dass α-Galaktosidasen von Cucumis melo hocheffizient α-Gal-Epitope auf der Oberfläche des Gewebes entfernen können, und dies deutlich besser als α-Galaktosidasen der grünen Kaffeebohne.

Figur 2 zeigt zusätzlich zu den obigen Daten die relative Leistungsfähigkeit einer α-Galaktosidase von Aspergillus niger. Wieder sind die Vergleichswerte von M86 initial, Native und Decellularized angegeben, wobei M86 initial wieder den Wert beschreibt, bei dem angenommen wird, dass keine α-Gal-Epitope mehr auf der Oberfläche des Gewebes vorhanden sind, während Native und Decellularized die Werte angeben von Gewebe, das nicht mit einer α-Galaktosidase behandelt wurde. Zu Grunde gelegt ist natives Gewebe bei einer Konzentration von 5 Units/ml. Es wird ersichtlich, wie auch schon aus Fig. 1, dass die α-Galaktosidase der grünen Kaffeebohne (GCB) in der Lage ist α-Gal-Epitope auf der Oberfläche des Gewebes zu entfernen. In Vergleich dazu zeigt sich jedoch, dass die saure α-Galaktosidase von Aspergillus niger (AN) bei dieser Konzentration kaum in der Lage ist α-Gal-Epitope auf der Oberfläche des Gewebes zu entfernen.

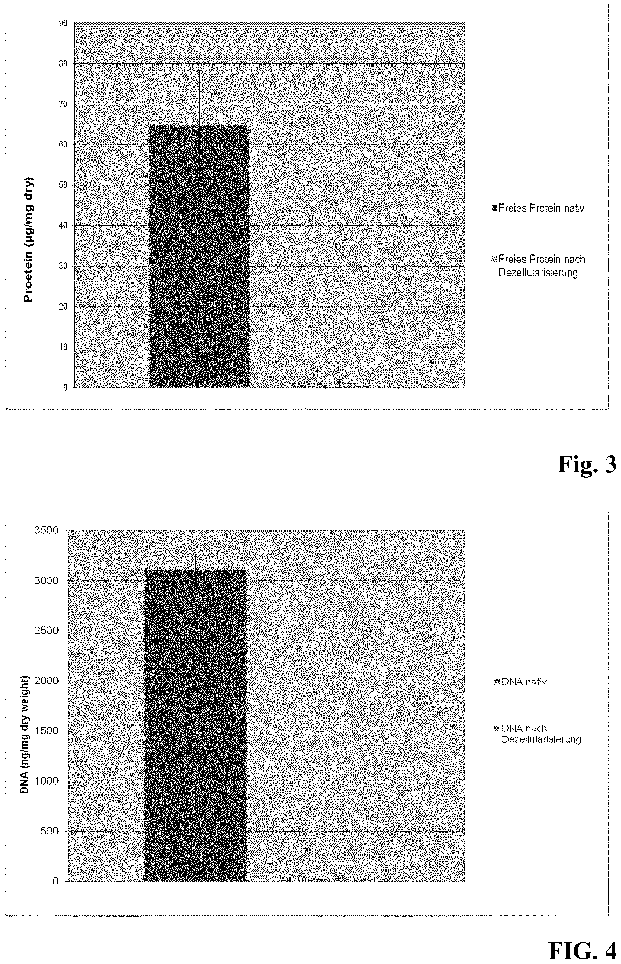

Figur 3 zeigt den Einfluss der Dezellularisierung mit Surfactin mit einer Konzentration von 0,06% in DPBS ohne Ca²⁺/Mg²⁺ innerhalb von 20 Stunden auf den Proteingehalt von porcinem Perikard. Wie Figur 3 zeigt, führt die Dezellularisierung zu einer signifikanten Abnahme von Proteinen.

In Figur 4 ist analog zu Figur 3 der Einfluss der unter Figur 3 beschriebenen Dezellularisierung auf den DNA-Gehalt von porcinem Perikard dargestellt. Die Dezellularisierung führt zu einer signifikanten Abnahme von DNA-Molekülen.

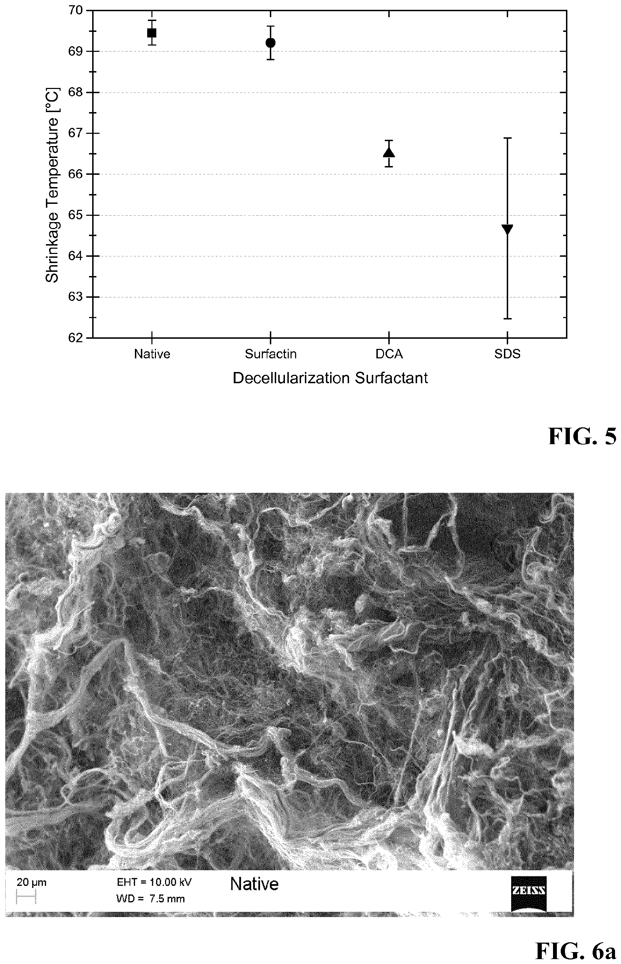

Figur 5 zeigt auf der Ordinate (vergrößerte Skala, Nullpunkt nicht abgebildet) die Schrumpfungstemperatur (shrinkage temperature) des dezellularisierten Gewebes nach Behandlung mit den drei Detergenzien Surfactin, Desoxycholsäure (DCA) und Natriumdodecylsulfat (SDS) im Vergleich zur Schrumpfungstemperatur des nativen Gewebes.

Aufgrund des dominierenden Anteils von Kollagen in der extrazellulären Matrix von Herzbeutelgewebe ist die Schrumpfungstemperatur die Temperatur, bei der das Protein Kollagen thermisch denaturiert, d.h. irreversibel seine räumliche Struktur verändert. Infolge der Strukturänderung der Kollagenmoleküle kommt es zu massiven irreversiblen Strukturänderungen im Gewebe, das bei Erreichen der Schrumpfungstemperatur deutlich sichtbar kleiner wird.

Experimentell bestimmt wurde die Schrumpfungstemperatur mittels Differential Scanning Calorimetry (DSC). Bei dieser Methode wird die Temperatur der zu vermessenden Probe linear mit der Zeit erhöht und der Wärmefluss in die bzw. aus der Probe bezüglich einer Referenzprobe vermessen. Kommt es zu thermodynamischen Prozessen in der Probe, z.B. der irreversiblen Strukturänderung des Kollagens, tritt im gemessenen Thermogramm ein deutlicher Peak bei der Schrumpfungstemperatur auf. Die Höhe der Schrumpfungstemperatur ist ein direkter Indikator für die Stabilität der räumlichen Struktur der Kollagenmoleküle. Eine möglichst geringe Veränderung gegenüber dem Zustand in nativem Gewebe ist deshalb ein direkter Beweis auf molekularer Ebene für die deutlich schonendere Dezellularisierung durch Surfactin.

Wie Figur 5 deutlich zeigt, ist die Schrumpfungstemperatur des Herzbeutelgewebes nach einer Dezellularisierung mit einem hierin beschriebenen Lipopeptid nahezu identisch der Schrumpfungstemperatur des unbehandelten nativen Herzbeutelgewebes. Die Dezellularisierung nach den beiden Ausführungsbeispielen mit DCA und SDS führen dagegen zu einer deutlich um 3°C bzw. 5°C reduzierten Schrumpfungstemperatur und damit einer deutlich beeinträchtigten Gewebestruktur. Die mechanischen Eigenschaften des nativen biologischen Gewebes und des Gewebes nach der Dezellularisierung sind daher sehr ähnlich. Die Dezellularisierung erfolgt somit nachweislich sehr schonend und kann ideal für das erfindungsgemäße Verfahren verwendet werden.

Die unterschiedliche Beeinträchtigung der Gewebestruktur zeigen auch die, in den Figuren 6a-d dargestellten, elektronenmikroskopischen Aufnahmen des nativen Gewebes bzw. des Gewebes nach Dezellularisierung mit den hierin genannten Detergenzien.

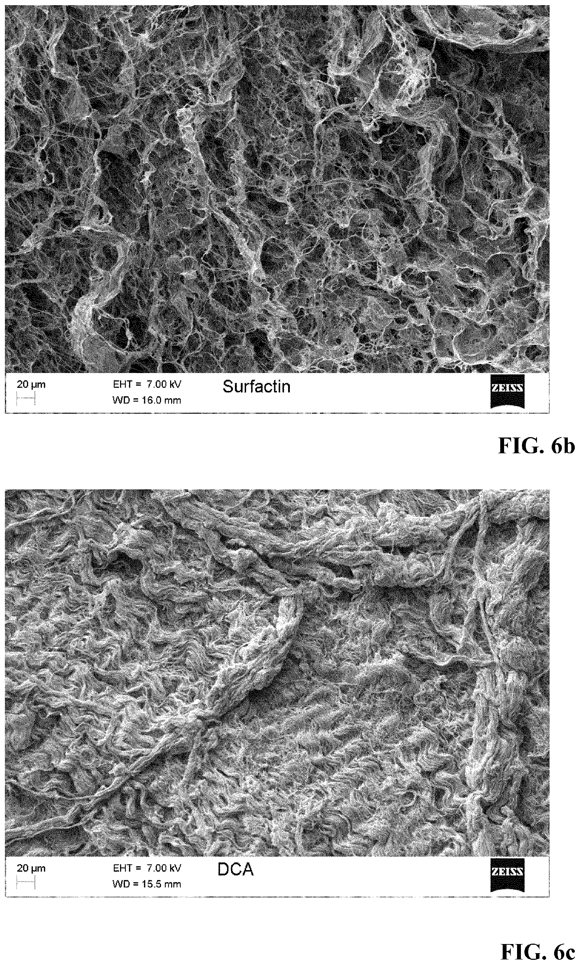

Beim Vergleich des nativen Gewebes in Figur 6a mit dem mit einem hierin beschriebenen Detergens dezellularisierten Gewebe in Figur 6b zeigt sich eine große Ähnlichkeit der Aufnahmen. Beide Gewebe zeigen viele voneinander getrennte Kollagenfasern und - stränge.

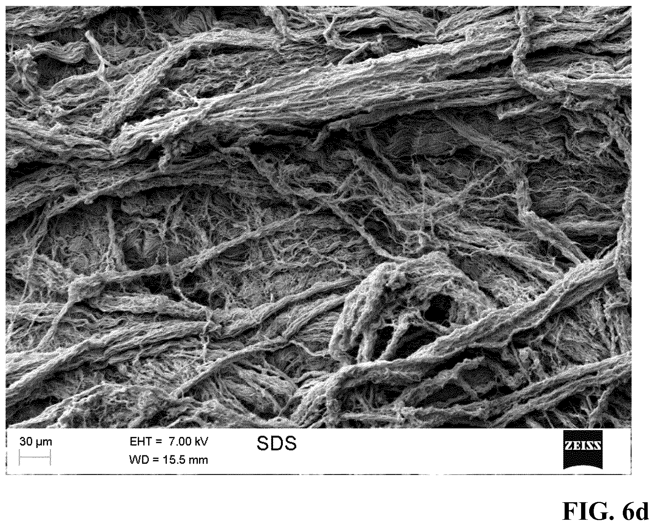

Im Vergleich dazu ist das in den Figuren 6c und 6d gezeigte Gewebe nach Dezellularisierung mit den genannten Detergenzien nach dem Stand der Technik deutlich verändert. Insbesondere kleinere Kollagenfaser neigen hier dazu, sich aneinander anzulagern. Dadurch wird die Gewebestruktur deutlich verändert und erscheint in den elektronenmikroskopischen Aufnahmen deutlich kompakter.

Im Folgenden wird eine Ausgestaltung eines vollständigen Verfahrens zur Aufbereitung von biologischem Gewebe für Implantatanwendungen nach dem vorliegenden Vorschlag in 12 Schritten detailliert beschrieben.

In Schritt 1 wird ein Herzbeutel einem Schwein im Schlachthof entnommen und für 2 Stunden bei einer Temperatur von 4°C in einer sterilen isotonischen Natriumchloridlösung (9 g/l; Fa. Fresenius-Kabi) gelagert. Die Lösung enthält neben Natriumchlorid auch Penicillin und/oder Streptomycin zum Abtöten bakterieller Keime.

In Schritt 2 wird das Gewebe feucht in einer Natriumchloridlösung (9 g/l; Fa. Fresenius-Kabi) präpariert. Das heißt, hier werden die beiden Blätter des Perikards voneinander getrennt, es wird anhaftendes Fett- und Bindegewebe vorsichtig entfernt und das Gewebe in Größe und Form auf die gewünschte Anwendung zugeschnitten.

Nach dem Spülen mit einer Natriumchloridlösung (9 g/l; Fa. Fresenius-Kabi) unter leichter Bewegung des Gewebes in Schritt 3 wird das Gewebe in Schritt 4 dezellularisiert.

Die Dezellularisierung in Schritt 4 erfolgt mit einem Detergens, welches aus einer Surfactin enthaltenden Pufferlösung besteht. In diesem Ausführungsbeispiel der Erfindung wird Surfactin (Fa. Sigma-Aldrich, Surfactin from Bacillus subtilis, Art.-Nr. F3523) mit einer Konzentration von 600 mg/l in einer DPBS Phosphatpufferlösung (Fa. Lonza; DPBS w/o Ca++/Mg++; Art.-Nr. 17-512) gelöst. Das Gewebe verbleibt für 20 Stunden bei 37°C in dieser Waschlösung. Anschließend ist das Gewebe nahezu vollständig von darin befindlichen Zellbestandteilen gereinigt, ohne das dabei die Struktur der Kollagenfasern wesentlich verändert wurde.

In Schritt 5 wird das Gewebe in 100 ml Natriumchloridlösung (9 g/l; Fa. Fresenius-Kabi) bei Raumtemperatur unter leichter Bewegung gespült. Schritt 5 wird dabei in diesem Ausführungsbeispiel der Erfindung 10 Minuten lang durchgeführt und 8-mal wiederholt.

Anschließend wird das Gewebe in Schritt 6 mit α-Galaktosidase von Cucumis melo mit einer Konzentration von 1 Unit pro ml (1 U/ml) in DPBS bei Raumtemperatur und einem pH von 7,4 für 24 Stunden behandelt und anschließend mit 200 ml DPBS gespült. Der Spülvorgang wird dabei sechs Mal wiederholt. Die α-Galaktosidase von Cucumis melo wurde bei Sigma Aldrich kommerziell erworben.

In Schritt 7 wird das Gewebe für 10 Minuten bei 37°C mit 100 ml einer 70%igen Ethanollösung gespült. In Schritt 8 schließt sich ein weiterer Spülschritt in 100 ml Natriumchloridlösung (9 g/l; Fa. Fresenius-Kabi) unter leichter Bewegung an.

In Schritt 9 erfolgt die Vernetzung der Kollagenfasern mit einem Vernetzungsmittel. In diesem Ausführungsbeispiel der Erfindung wird das Gewebe für 48 Stunden bei einer Temperatur von 4°C in eine Glutaraldehyd (Fa. Sigma-Aldrich, Art.-Nr. F5882) enthaltende Lösung bei pH 7.4 gelegt. Die Glutaraldehyd enthaltende Lösung besteht aus Glutaraldehyd mit einer Konzentration von 6 g/l in DPBS ohne Kalzium und Magnesium (Fa. Lonza; DPBS w/o Ca++/Mg++; Art.-Nr. 17-512).

Schritt 10 wiederholt Schritt 9 bei Raumtemperatur. Schritt 10 wird 14 Tage lang durchgeführt, wobei die Lösung alle 48 Stunden ausgetauscht wird.

In Schritt 11 wird das Gewebe in diesem Ausführungsbeispiel der Erfindung 6-mal für 20 Minuten bei Raumtemperatur unter leichter Bewegung mit 100 ml Natriumchloridlösung (9 g/l; Fa. Fresenius-Kabi) gespült. Nach einem Spülprozess in Schritt 11 kann das Gewebe in Glutaraldehyd gelagert oder in Schritt 12 weiter verarbeitet werden.

Das hier geschilderte Ausführungsbeispiel dient der Verdeutlichung der Erfindung. Zahl und/oder Ausgestaltung der Spülschritte (insbesondere Konzentrationen, Dauer, Temperaturen und Zusammensetzung der Lösung zum Spülen oder der Pufferlösung) können vom Fachmann im Rahmen seiner Kenntnisse variiert werden.

## Patentansprüche

1. Verfahren zur Aufbereitung von Gewebe für medizinische Anwendungen, insbesondere von Gewebe zur Verwendung für eine künstliche Herzklappe, wobei das Verfahren zumindest folgende Schritte umfasst:
Dezellularisierung des Gewebes mittels eines Detergens,
Behandlung des Gewebes mit einer α-Galaktosidase, und
Vernetzung der Kollagenfasern des Gewebes mittels eines geeigneten Vernetzungsmittels, **dadurch gekennzeichnet dass** die α-Galaktosidase von Cucumis Melo stammt.

2. Verfahren nach Anspruch 1, **dadurch gekennzeichnet, dass** das Detergens zur Dezellularisierung mindestens ein Lipopeptid mit amphiphilen Eigenschaften, bestehend aus einer hydrophilen Grundstruktur und einer hydrophoben Seitenkette, enthält.

3. Verfahren nach einem der vorangegangenen Ansprüche, **dadurch gekennzeichnet, dass** das Detergens zur Dezellularisierung Surfactin, Daptomycin, Caspofungin, Arthrofactin, ein Echinocandin, ein Iturin, ein Syringomycin, ein Syringopeptid und/oder ein Polymyxin enthält.

4. Verwendung einer α-Galaktosidase zur Behandlung von biologischem Gewebe für Herzklappenprothesen, **dadurch gekennzeichnet, dass** die α-Galaktosidase von Cucumis Melo stammt.

## Claims

1. A process for preparing tissue for medical applications, in particular tissue for use for an artificial heart valve, this process comprising the following steps:
decellularizing the tissue by means of a detergent;
treating the tissue with an α-galactosidase; and
cross-linking the collagen fibers of the tissue by means of a suitable cross-linking agent, **characterized in that** the α-galactosidase comes from *Cucumis melo.*

2. A process according to claim 1, **characterized in that** the detergent for decellularization contains at least one lipopeptide with amphiphilic properties, consisting of a hydrophilic basic structure and a hydrophobic side chain.

3. A process according to any one of the preceding claims, **characterized in that** the detergent for decellularization contains surfactin, daptomycin, caspofungin, arthrofactin, an echinocandin, an iturin, a syringomycin, a syringopeptide , and/or a polymyxin.

4. The use of an α-galactosidase for treatment of biological tissue for heart valve prostheses, **characterized in that** the α-galactosidase comes from *Cucumis melo.*

## Revendications

1. Procédé de préparation de tissus pour des applications médicales, notamment de tissus pour l'utilisation en tant que valvule cardiaque artificielle, où le procédé comprend au moins les étapes suivantes:
la décellularisation du tissu au moyen d'un détergent,
le traitement du tissu avec une α-galactosidase, et
le mouillage des fibres de collagène du tissu au moyen d'un produit mouillant approprié, **caractérisé en ce que** la α-galactosidase provient du melon.

2. Procédé selon la revendication 1, **caractérisé en ce que** le détergent pour la décellularisation contient un lipopeptide doté de propriétés amphiphiles, constitué d'une structure de base hydrophile et d'une chaîne latérale hydrophobe.

3. Procédé selon l'une des revendications précédentes, **caractérisé en ce que** le détergent pour la décellularisation contient de la surfactine, de la daptomycine, de la caspofungine, de l'arthrofactine, une échinocandine, une iturine, une syringomycine, un syringopeptide et/ou une polymyxine.

4. Utilisation d'une α-galactosidase pour le traitement de tissus biologiques destinés à des prothèses de valvules cardiaques, **caractérisée en ce que** l'a-galactosidase provient du melon.
